# EUROPEAN PATENT APPLICATION

(11) **EP 2 517 707 A1**
(43) Date of publication of application: **31.10.2012**
(21) Application number: 10839541.9
(22) Date of filing: 24.12.2010
(51) Int. Cl.: A61K 31/404, A61P 35/00, C07D 403/06

(54) **ANTITUMOR AGENT OR POSTOPERATIVE ADJUVANT CHEMOTHERAPEUTIC AGENT FOR TREATMENT OF HEPATOCELLULAR CARCINOMA**

(30) Priority: 25.12.2009 JP 2009295909
(71) Applicant: Taiho Pharmaceutical Co., Ltd., Chiyoda-ku, Tokyo 101-8444 (JP)
(72) Inventor: OKA Toshinori, Tokushima-shi Tokushima 771-0194 (JP)
(74) Representative: Schrell, Andreas
(86) International application number: PCT/JP2010/073298
(87) International publication number: WO 2011/078311

(57) **Abstract**

The present invention provides an antitumor agent or postoperative adjuvant chemotherapeutic agent for a hepatocellular carcinoma patient who has been treated by transarterial embolization. An antitumor agent or postoperative adjuvant chemotherapeutic agent comprising TSU-68 or a salt thereof, which can be administered to a hepatocellular carcinoma patient within 2 weeks after transarterial embolization, is provided.

## Description

### Technical Field

The present invention relates to an antitumor agent or postoperative adjuvant chemotherapeutic agent used for treatment of a hepatocellular carcinoma patient who has been treated with transarterial embolization. Specifically, the present invention relates to an antitumor agent or postoperative adjuvant chemotherapeutic agent that can be administered to a hepatocellular carcinoma patient who has been treated with transarterial embolization within 2 weeks after transarterial embolization.

### Background Art

There are a variety of hepatocellular carcinoma treatment methods such as surgical resection, transarterial embolization (hereinafter referred to as "TAE"), percutaneous ablation therapy (PAT) (e.g., percutaneous ethanol injection therapy (PEIT) or radiofrequency ablation (RFA)), chemotherapy, radiation therapy, and liver transplantation. TAE is a surgical treatment method comprising injecting an embolic material such as gelatin sponge into the hepatic artery of a hepatocellular carcinoma patient to embolize the nutrient artery so as to cause necrosis of hepatocellular carcinoma in a selective manner. TAE also includes transcatheter arterial chemo-embolization (hereinafter referred to as "TACE") for administering an antitumor agent to lesions during surgical treatment. TAE plays an important role in hepatocellular carcinoma treatment because TAE is the most frequently used treatment method for initial treatment cases and recurring cases, and TAE is particularly recommended for cases in which surgical resection or PAT is unavailable as well as recurring cases (Non-Patent Literature 1). Meanwhile, TAE is problematic in that TAE rarely results in complete tumor necrosis after treatment, and thus, in many cases, tumor remains in the peripheral zone of the treatment site, causing recurrence or metastasis several months after TAE. Thus, if inhibition of such recurrence or metastasis becomes possible, it can contribute to further extension of life expectancy.

Hitherto, there have been attempts to develop a variety of antitumor agents for hepatocellular carcinoma. For example, there are anthracycline antitumor agents, platinum antitumor agents, alkaloid antitumor agents, nucleoside antimetabolites, and the like. However, it is said that administration of such antitumor agents is not recommended according to treatment guidelines (Non-Patent Literature 1 and Non-Patent Literature 2). The above antitumor agents also can be used as chemotherapeutic agents for TACE. However, no excellent drugs that can be recommended for TACE in particular have been found. In addition, it was even reported that there are no obvious differences between TAE and TACE in terms of antitumor effects (Non-Patent Literature 3).

In recent years, the elucidation of cancer at the molecular biological level has led to discovery of a variety of molecular-targeted antitumor agents that target specific molecules such as angiogenesis-related factors and cell-growth-related factors expressed in cancer cells. Such molecular-targeted antitumor agents differ from conventional antitumor agents in terms of action mechanisms and toxicity profiles. Thus, they have been gaining attention because of their potential to contribute to treatment of hepatocellular carcinoma.

(Z)-5-[(1,2-dihydro-2-oxo-3H-indol-3-ylidene)methyl]2,4-dimethyl-1H-pyrrol-3-propanoic acid (hereinafter referred to as "TSU-68"), which is known as a molecular-targeted antitumor agent, is a low-molecular-weight compound. It inhibits tyrosine phosphorylation of Flk-1 (also referred to as "KDR"), which is a vascular endothelial growth factor (hereinafter referred to as "VEGF") receptor, so as to inhibit angiogenesis in tumor tissue for interruption of oxygen and nutrition supply, thereby inhibiting tumor growth and metastasis. In addition, it has been confirmed *in vitro* that the low-molecular-weight compound also inhibits tyrosine phosphorylation of platelet-derived growth factor (hereinafter referred to as "PDGF") receptors, FGF receptors, and the like, which are involved in intracellular signal transduction, as well as the VEGF receptors. As a result of examination of antitumor effects of the oral administration of TSU-68 alone upon *in vivo* nude mouse models into which various human cancer cell lines had been subcutaneously implanted, TSU-68 was confirmed to be effective for inhibiting tumor growth in lung cancer, large bowel cancer, uterus cancer, and the like (Non-Patent Literature 4). It has been reported that TSU-68 has treatment effects on some types of cancer such as hepatocellular carcinoma in clinical studies (Non-Patent Literature 5).

In addition to TSU-68, there are known VEGF receptor inhibitors such as sunitinib and sorafenib. There have been attempts to conduct clinical studies to use sunitinib and sorafenib for adjuvant therapy after TAE; however, it still has not been reported whether treatment effects have been confirmed (Non-Patent Literature 6).

It has been reported that the hyperexpression of angiogenesis factors such as VEGF is transiently observed in hepatocellular carcinoma starting from the day after TAE (Non-Patent Literature 7, Non-Patent Literature 8, and Non-Patent Literature 9). This indicates hypoxic response as a result of tumor necrosis caused by TAE. Such hyperexpression of the angiogenesis factors is considered to be a cause of metastasis/recurrence. However, it is also known that TAE negatively influences liver function, while TAE is expected to be effective against cancer cells. It was reported that not only the levels of liver disorder markers such as bilirubin and aspartate aminotransferase (AST) but also the Child Pugh score used as a comprehensive assessment parameter for liver function disorder significantly increase after TAE to higher levels than before TAE (Non-Patent Literature 10 and Non-Patent Literature 11). As a result of clinical studies of sorafenib for unresectable hepatocellular carcinoma patients, sorafenib was found to increase the bilirubin level (corresponding to score 3 or more) and the AST level to 32% and 45%, respectively, in patients, causing deterioration in liver function (Non-Patent Literature 12).

In view of the above, antitumor agent administration soon after TAE is not preferable because it might cause further liver function decline, which would disadvantageously result in increased incidence of adverse reactions.

As described above, no treatment method has been established as a method that should be used as standard therapy in the hepatocellular carcinoma treatment system. Also, efficacy of adjuvant chemotherapy performed after TAE has not been established. Therefore, drugs that can be administered soon after TAE have been particularly awaited.

### Citation List

### Non-Patent Literature

Non-Patent Literature 1: National Comprehensive Cancer Network Clinical Practice Guidelines in Oncology Hepatobiliary Cancers v.2, 2009
Non-Patent Literature 2: Ann Surg Oncol 15: 1008, 2008
Non-Patent Literature 3: Expert Rev Anticancer Ther 8: 1623, 2008
Non-Patent Literature 4: Cancer Res 60: 4152, 2008
Non-Patent Literature 5: Euro J Cancer Suppl 6(12): 17 #43, 2008
Non-Patent Literature 6: BMC Cancer 8: 349, 2008
Non-Patent Literature 7: Int J Oncol 14: 1087-1090, 1999
Non-Patent Literature 8: J Huazhong Univ Sci Technolog Med Sci 27: 58, 2007
Non-Patent Literature 9: Cancer Sci 99: 2037, 2008
Non-Patent Literature 10: Radiology 215: 123, 2000
Non-Patent Literature 11: Hepatogastroenterology 54: 1499, 2007
Non-Patent Literature 12: Cancer 115: 428, 2009

### Summary of Invention

### Technical Problem

An object of the present invention is to provide an antitumor agent or postoperative adjuvant chemotherapeutic agent that is used to treat a hepatocellular carcinoma patient who has been treated with TAE.

### Solution to Problem

The present inventors conducted intensive studies to develop a cancer treatment method that contributes to the improvement of treatment effects and, in particular, life-extending effects for patients. As a result, the present inventors have found that excellent treatment effects can be obtained for treatment of a hepatocellular carcinoma patient by administering TSU-68 to the patient soon after TAE. This has led to the completion of the present invention.

Specifically, the present invention relates to the following inventions [1] to [12].

[1] An antitumor agent comprising (2)-5-[(1,2-dihydro-2-oxo-3H-indol-3-ylidene)methyl]2,4-dimethyl-1H-pyrro 1-3-propanoic acid or a salt thereof, which is used to initiate administration of
(Z)-5-[(1,2-dihydro-2-oxo-3H-indol-3-ylidene)methyl]2,4-dimethyl-1H-pyrro 1-3-propanoic acid or a salt thereof within 2 weeks after transarterial embolization to a hepatocellular carcinoma patient who has been treated with transarterial embolization for treatment of the patient.

[2] The antitumor agent according to [1], wherein transarterial embolization is transarterial chemoembolization.

[3] A kit including the antitumor agent of [1] or [2] and instructions for use that specify initiation of administration of (Z)-5-[(1,2-dihydro-2-oxo-3H-indol-3-ylidene)methyl]2,4-dimethyl-1H-pyrro 1-3-propanoic acid or a salt thereof within 2 weeks after transarterial embolization to a hepatocellular carcinoma patient who has been treated with transarterial embolization.

[4] A postoperative adjuvant chemotherapeutic agent comprising (Z)-5-[(1,2-dihydro-2-oxo-3H-indol-3-ylidene)methyl]2,4-dimethyl-1H-pyrro 1-3-propanoic acid or a salt thereof, which is used to initiate administration of
(Z)-5-[(1,2-dihydro-2-oxo-3H-indol-3-ylidene)methyl]2,4-dimethyl-1H-pyrro 1-3-propanoic acid or a salt thereof within 2 weeks after transarterial embolization to a hepatocellular carcinoma patient who has been treated with transarterial embolization for treatment of hepatocellular carcinoma.

[5] The postoperative adjuvant chemotherapeutic agent of [4], wherein transarterial embolization is transarterial chemoembolization.

[6] A kit including the postoperative adjuvant chemotherapeutic agent of [4] or [5] and instructions for use that specify initiation of administration of
(Z)-5-[(1,2-dihydro-2-oxo-3H-indol-3-ylidene)methyl]2,4-dimethyl-1H-pyrro 1-3-propanoic acid or a salt thereof within 2 weeks after transarterial embolization to a hepatocellular carcinoma patient who has been treated with transarterial embolization.

[7] A method for treating hepatocellular carcinoma of a hepatocellular carcinoma patient who has been treated with transarterial embolization, which comprises initiating administration of an antitumor agent comprising (Z)-5-[(1,2-dihydro-2-oxo-3H-indol-3-ylidene)methyl]2,4-dimethyl-1H-pyrro 1-3-propanoic acid or a salt thereof to the hepatocellular carcinoma patient within 2 weeks after transarterial embolization.

[8] The method of [7], wherein transarterial embolization is transarterial chemoembolization.

[9] A method for treating hepatocellular carcinoma of a hepatocellular carcinoma patient who has been treated with transarterial embolization, which comprises administering a postoperative adjuvant chemotherapeutic agent comprising
(Z)-5-[(1,2-dihydro-2-oxo-3H-indol-3-ylidene)methyl]2,4-dimethyl-1H-pyrro 1-3-propanoic acid or a salt thereof to the hepatocellular carcinoma patient within 2 weeks after transarterial embolization.

[10] The method of [9], wherein transarterial embolization is transarterial chemoembolization.

[11] (Z)-5-[(1,2-dihydro-2-oxo-3H-indol-3-ylidene)methyl]2,4-dimethyl-1H-pyrrol-3-propanoic acid or a salt thereof, which is used for a hepatocellular carcinoma patient who has been treated with transarterial embolization within 2 weeks after transarterial embolization for treatment of hepatocellular carcinoma.

[12] The
(Z)-5-[(1,2-dihydro-2-oxo-3H-indol-3-ylidene)methyl]2,4-dimethyl-1H-pyrro 1-3-propanoic acid or a salt thereof of [11], wherein transarterial embolization is transarterial chemoembolization.

This description includes part or all of the contents as disclosed in the description and/or drawings of Japanese Patent Application No. 2009-295909, which is a priority document of the present application. Advantageous Effects of Invention

The present invention can extend progression-free survival through treatment of hepatocellular carcinoma patients after TAE, for which no standard therapy has been established. In addition, excellent treatment effects can be provided with no increase in the incidence of adverse reactions. Further, according to the present invention, antitumor agents or postoperative adjuvant chemotherapeutic agents can be administered to hepatocellular carcinoma patients who have been treated with transarterial embolization soon after TAE. Thus, efficient hepatocellular carcinoma treatment or prevention of hepatocellular carcinoma recurrence can be achieved.

Antitumor agents and postoperative adjuvant chemotherapeutic agents for treating hepatocellular carcinoma patients after TAE, which are characterized by high degrees of treatment retention and treatment efficacy, were found for the first time based on the present invention.

### Description of Embodiments

The present invention relates to an antitumor agent and a postoperative adjuvant chemotherapeutic agent each comprising TSU-68 or a salt thereof. The "antitumor agent" used herein is a chemotherapeutic agent which is administered to treat a tumor. The "postoperative adjuvant chemotherapeutic agent" used herein is a chemotherapeutic agent which is administered to prevent tumor recurrence or metastasis after surgical treatment of tumor lesions by surgery or the like.

"TSU-68" is a known inhibitor of a VEGF receptor or the like denoted as (Z)-5-[(1,2-dihydro-2-oxo-3H-indol-3-ylidene)methyl]2,4-dimethyl-1H-pyrro 1-3-propanoic acid. TSU-68 is known to have tumor-growth-inhibiting effects upon solid cancers such as liver cancer, lung cancer, large bowel cancer, and uterus cancer. TSU-68 or a salt thereof can be produced by a known method, such as the method of JP Patent Publication (Kohyo) No. 2002-516310 A.

A salt of TSU-68 is not particularly limited as long as it is a pharmaceutically acceptable salt. Examples thereof include salts obtained by reacting TSU-68 with inorganic acids such as hydrochloric acid, hydrobromic acid, sulfuric acid, nitric acid, and phosphoric acid and organic acids such as methanesulfonic acid, ethanesulfonic acid, p-toluenesulfonic acid, and salicylic acid.

The administration form of an antitumor agent or postoperative adjuvant chemotherapeutic agent comprising TSU-68 or a salt thereof is not particularly limited, and thus it can be adequately selected depending on treatment purposes. Specific examples thereof include oral agents (e.g., tablets, coated tablets, powders, granules, capsules, and liquids), parenteral injections, suppositories, patches, and ointments. Oral agents are preferable.

The antitumor agent or postoperative adjuvant chemotherapeutic agent comprising TSU-68 or a salt thereof can be prepared by a conventionally known method using a pharmaceutically acceptable carrier. Examples of such carrier include a variety of carriers widely used for general drugs, such as excipients, binders, disintegrators, lubricants, diluents, solubilizing agents, suspending agents, isotonizing agents, pH adjusters, buffers, stabilizers, colorants, corrigents, and flavoring agents.

Examples of excipients include lactose, sucrose, sodium chloride, glucose, maltose, mannitol, erythritol, xylitol, maltitol, inositol, dextran, sorbitol, albumin, urea, starch, calcium carbonate, kaoline, crystalline cellulose, silicic acid, methylcellulose, glycerine, sodium alginate, gum arabic, and mixtures thereof. Examples of lubricants include purified talc, stearate, borax, polyethylene glycol, and mixtures thereof. Examples of binders include simple syrups, glucose solutions, starch solutions, gelatin solutions, polyvinyl alcohol, polyvinyl ether, polyvinyl pyrrolidone, carboxymethylcellulose, shellac, methylcellulose, ethylcellulose, water, ethanol, potassium phosphate, and mixtures thereof. Examples of disintegrators include dry starch, sodium alginate, agar powder, laminaran powder, sodium hydrogen carbonate, calcium carbonate, polyoxyethylene sorbitan fatty acid esters, sodium lauryl sulfate, stearic acid monoglyceride, starch, lactose, and mixtures thereof. Examples of diluents include water, ethyl alcohol, macrogol, propylene glycol, ethoxylatedisostearyl alcohol, polyoxygenated isostearyl alcohol, polyoxyethylene sorbitan fatty acid esters, and mixtures thereof. Examples of stabilizers include sodium pyrosulfite, ethylenediamine tetraacetate, thioglycollic acid, thiolactic acid, and mixtures thereof. Examples of isotonizing agents include sodium chloride, boric acid, glucose, glycerine, and mixtures thereof. Examples of pH adjusters and buffers include sodium citrate, citric acid, sodium acetate, sodium phosphate, and mixtures thereof. Examples of soothing agents include procaine hydrochloride, lidocaine hydrochloride, and mixtures thereof.

Patients for whom the present invention is used are hepatocellular carcinoma patients who have been treated with TAE. TAE is a surgical treatment method comprising injecting an embolic material such as gelatin sponge into the hepatic artery that supplies nutrition to tumor cells to embolize the nutrient artery so as to cause necrosis of hepatocellular carcinoma in a selective manner (National Comprehensive Cancer Network Clinical Practice Huidelines in Oncology Hepatobiliary Cancers v.2, 2009). In addition, TAE of the present invention also includes TACE for administration of antitumor agents. Examples of TACE include a method wherein a mixture of an oily contrast agent such as lipiodol and antitumor agent(s) is injected into the nutrient artery prior to injection of an embolic material and a method wherein drug (antitumor agent)-eluting beads are used instead of antitumor agents (i.e., DEB-TACE) (J Hepatol. 2007 Mar; 46(3): 474-81). Further, antitumor agents used for TACE are not particularly limited as long as they can be used for hepatocellular carcinoma. Examples thereof include doxorubicin, epirubicin, and cisplatin.

Patients for whom the present invention is used are particularly preferably patients with good hepatic functional reserve. Herein, hepatic functional reserve can be evaluated using any or a combination of the following examples of parameters: aspartate aminotransferase (also referred to as "GOT"), glutamic pyruvic transaminase (also referred to as "GPT"), γ-glutamyl transpeptidase (also referred to as "γ-GTP"), alkaline phosphatase (also referred to as "ALP"), HBs antigens, total protein, albumin, thymol turbidity test (also referred to as "TTT"), zinc sulfate turbidity test (also referred to as "ZTT"), creatine kinase (also referred to as "CPK"), albumin/globulin ratio (also referred to as "A/G ratio"), amylase, cholinesterase, leucine aminopeptidase (also referred to as "LAP"), lactate dehydrogenase (also referred to as "LDH"), bilirubin, albumin, prothrombin time (%) (also referred to as "PT"), encephalopathy, nutritional state, ascites, the degree of coma, and indocyanine green (ICG) study. Evaluation is preferably carried out by Child classification (Saunders Philadelphia (III ed) S50, 1965) or Child-Pugh classification (the General Rules for the Clinical and Pathological Study of Primary Liver Cancer, 4th edition, (November, 2000)) and further preferably by Child-Pugh classification. In addition, a good hepatic functional reserve state corresponds to Class A or B and more preferably Class A according to Child-Pugh classification, for example.

In general, an antitumor agent or postoperative adjuvant chemotherapeutic agent comprising TSU-68 or a salt thereof is administered alone. Alternatively, it can be administered in combination with a different antitumor agent.

When the antitumor agent or postoperative adjuvant chemotherapeutic agent is administered in combination with a different antitumor agent, the agents may be formulated into a combination drug (a formulation comprising a plurality of active ingredients) in a single dosage form (i.e., a single-dose formulation) or a combination of formulations in a plurality of dosage forms (i.e., a multiple-dose formulation), each of the formulations comprising a different single drug comprising any of the above active ingredients (a formulation comprising a single active ingredient), for simultaneous administration or separate administration at certain intervals. In addition, for production/packaging/distribution of an antitumor agent or postoperative adjuvant chemotherapeutic agent comprising TSU-68 or a salt thereof, each of the above formulations may be separately produced/packaged/distributed, or some or all of the formulations may be produced/packaged/distributed in a single package (kit formulation) appropriate for combined administration. In addition, in the case of formulation in a plurality of dosage forms, the formulations may be in different or uniform administration forms.

The administration schedule for the antitumor agent or postoperative adjuvant chemotherapeutic agent comprising TSU-68 or a salt thereof can be adequately determined depending on conditions such as patient age, sex, and weight, disease stage, the presence or absence of metastasis, and the history of treatment. However, administration is initiated within approximately 2 weeks such as within 14 days, 10 days, 5 days, 3 days, 2 days, or 1 day after TAE. Alternatively, administration and TAE can be carried out at the same time. It is known that the VEGF level in serum continues to increase transiently in hepatocellular carcinoma patients within 2 weeks after TAE, and that a larger increase in the VEGF level in a patient indicates poorer prognosis for the patient. Therefore, it is preferable to initiate administration of the antitumor agent or postoperative adjuvant chemotherapeutic agent comprising TSU-68 or a salt thereof within a period during which the VEGF level increases to a greater extent (i.e., approximately within 2 weeks after TAE). Hitherto, administration of the antitumor agent or postoperative adjuvant chemotherapeutic agent to the liver after TAE was impossible in the early stage after TAE (e.g., within approximately 1 month, preferably approximately 3 weeks, and more preferably approximately 2 weeks after TAE) in consideration of TAE-related burden on the liver and the increase in the incidence of adverse reactions associated with such burden. Meanwhile, the antitumor agent or postoperative adjuvant chemotherapeutic agent of the present invention can be administered in the early stage after TAE without inducing adverse reactions, thereby advantageously allowing efficient treatment of hepatocellular carcinoma and recurrence of hepatocellular carcinoma as described in detail in the Examples below.

The administration dose of an antitumor agent or postoperative adjuvant chemotherapeutic agent comprising TSU-68 or a salt thereof can be adequately determined depending on conditions such as patient age, sex, and weight, disease stage, the presence or absence of metastasis, the history of treatment, and the use or non-use of a different antitumor agent. For example, it is preferably 100 to 3000 mg/day, more preferably 200 to 1600 mg/day, and particularly preferably 400 to 800 mg/day. In addition, the administration schedule of the antitumor agent can be adequately determined depending on conditions such as patient age, sex, and weight, disease stage, the presence or absence of metastasis, and the history of treatment. For example, it is preferable to administer the antitumor agent once daily or two to four times daily in separate doses on consecutive days.

The present invention also relates to an antitumor agent kit for treating hepatocellular carcinoma of a hepatocellular carcinoma patient who has been treated with TAE. The antitumor agent kit includes an antitumor agent comprising TSU-68 or a salt thereof and instructions for use that specify administration of the antitumor agent comprising TSU-68 or a salt thereof within 2 weeks after TAE. The present invention further relates to a postoperative adjuvant chemotherapeutic agent kit for treating hepatocellular carcinoma of a hepatocellular carcinoma patient who has been treated with TAE. The postoperative adjuvant chemotherapeutic agent kit includes a postoperative adjuvant chemotherapeutic agent comprising TSU-68 or a salt thereof and instructions for use that specify administration of the postoperative adjuvant chemotherapeutic agent comprising TSU-68 or a salt thereof within 2 weeks after TAE. Here, "instructions for use" are not legally binding as long as they contain descriptions of TSU-68 administration methods/administration criteria. Specific examples thereof include package inserts and pamphlets. In addition, "instructions for use" may be printed on or attached to a package for an antitumor agent kit or a postoperative adjuvant chemotherapeutic agent kit or may be enclosed together with an antitumor agent or a postoperative adjuvant chemotherapeutic agent in the package for an antitumor agent kit or a postoperative adjuvant chemotherapeutic agent kit.

The present invention relates to a method for treating hepatocellular carcinoma in a hepatocellular carcinoma patient who has been treated with TAE by administering an antitumor agent comprising TSU-68 or a salt thereof within 2 weeks after TAE.

The present invention further relates to a method for preventing hepatocellular carcinoma recurrence or metastasis in a hepatocellular carcinoma patient who has been treated with TAE by administering a postoperative adjuvant chemotherapeutic agent comprising TSU-68 or a salt thereof within 2 weeks after TAE.

### [Examples]

The present invention is hereafter described in greater detail with reference to the following examples, although the scope of the present invention is not limited thereto.

### TSU-68 therapy for hepatocellular carcinoma patients treated with TACE

A comparative clinical study was conducted for the TSU-68 administration group and the TSU-68 non-administration group consisting of cases that were impossible to treat with hepatectomy and PAT and had been treated with TACE and excluding cases evaluated as Child-Pugh grade C in terms of hepatic functional reserve. The antitumor agent used for TACE was epirubicin.

The subject cases were randomly assigned to the TSU-68 administration group and the TSU-68 non-administration group. TSU-68 administration was initiated for cases assigned to the TSU-68 administration group within 2 weeks after TACE. TSU-68 was administered orally at a dose of 200 mg per administration twice daily after breakfast and dinner on consecutive days. No further treatment was given to the TSU-68 non-administration group after TACE and thus only follow-up observation was carried out.

Progression-free survival was defined as survival within the period from the day of TACE designated as the initial date of reckoning to any of dates on which the following events was observed, whichever came first.

1. Date on which the presence of progressive disease (hereinafter referred to as "PD") was confirmed as a result of assessment of best overall response (date of testing).

2. Date on which a test subject died for any reason (date of death).

3. Date on which a test subject was assessed to have experienced clinically obvious deterioration of the disease or to be in need of post-TACE treatment even without confirmation of PD upon assessment of best overall response (date of assessment).

Best overall response was evaluated by measuring tumor diameters by CT and assaying tumor markers (AFP, PIVKA-II, and AFP-L3 fractions) according to New Guideline to Evaluate the Response to Treatment in Solid Tumor (hereinafter referred to as "RECIST") (J Nat1 Cancer Inst 92: 205, 2000).

Table 1 shows treatment outcomes for the TSU-68 administration group and the TSU-68 non-administration group consisting of hepatocellular carcinoma patients treated with TACE in this study.

**[Table 1]**

| Treatment outcomes for the TSU-68 administration group and the TSU-68 non-administration group of post-TACE hepatocellular carcinoma patients | | |
|---|---|---|
| Subject | Number of cases | Median of progression-free survival (month) |
| TSU-68 administration group | 50 | 5.2 |
| TSU-68 non-administration group | 51 | 4.0 |

In addition, table 2 shows treatment outcomes for the TSU-68 administration group and the TSU-68 non-administration group consisting of TACE-treated hepatocellular carcinoma patients who had been diagnosed as Child-Pugh classification A in terms of hepatic functional reserve in this study.

**[Table 2]**

| Treatment outcomes for the TSU-68 administration group and the TSU-68 non-administration group consisting of TACE-treated hepatocellular carcinoma patients classified as grade A according to Child-Pugh classification | | |
|---|---|---|
| Subject | Number of cases | Median of progression-free survival (month) |
| TSU-68 administration group | 36 | 5.8 |
| TSU-68 non-administration group | 41 | 4.0 |

As a result, the median of progression-free survival for the TSU-68 administration group and was 5.2 months and 4.0 months for the TSU-68 non-administration group, each of such groups consisting of TACE-treated hepatocellular carcinoma patients. Thus, it was confirmed that progression-free survival is significantly extended for the TSU-68 administration group. In addition, the median for progression-free survival was 5.8 months for the TSU-68 administration group and 4.0 months for the TSU-68 non-administration group, each of such groups consisting of TACE-treated hepatocellular carcinoma patients who had been diagnosed as Child-Pugh grade A in terms of hepatic functional reserve. Thus, it was confirmed that progression-free survival is significantly extended for the TSU-68 administration group. In addition, major adverse reactions were abdominal pain, ascites, edema, general fatigue, fever, and a feeling of sickness. The severity of each adverse reaction corresponded to grade 1 or 2 within the acceptable range. Continuous administration of TSU-68 soon after TACE probably resulted in inhibition of angiogenesis due to excessive increase in the VEGF level soon after TACE, contributing to an extension of progression-free survival without serious adverse reactions.

As described above, it was revealed that excellent treatment effects can be provided through administration of TSU-68 to hepatocellular carcinoma patients who have been treated with TACE soon after TACE.

### Industrial Applicability

According to the present invention, chemotherapy can be started for hepatocellular carcinoma patients soon after TACE, making it possible to efficiently treat hepatocellular carcinoma and/or prevent recurrence of hepatocellular carcinoma after TACE. Thus, the method of the present invention can be expected as a novel therapy.

All publications, patents, and patent applications cited herein are incorporated herein by reference in their entirety.

## Claims

1. An antitumor agent comprising (Z)-5-[(1,2-dihydro-2-oxo-3H-indol-3-ylidene)methyl]2,4-dimethyl-1H-pyrro 1-3-propanoic acid or a salt thereof, which is used to initiate administration of
(Z)-5-[(1,2-dihydro-2-oxo-3H-indol-3-ylidene)methyl]2,4-dimethyl-1H-pyrro 1-3-propanoic acid or a salt thereof within 2 weeks after transarterial embolization to a hepatocellular carcinoma patient who has been treated with transarterial embolization for treatment of hepatocellular carcinoma.

2. The antitumor agent according to claim 1, wherein transarterial embolization is transarterial chemoembolization.

3. A kit including the antitumor agent of claim 1 or 2 and instructions for use that specify initiation of administration of (Z)-5-[(1,2-dihydro-2-oxo-3H-indol-3-ylidene)methyl]2,4-dimethyl-1H-pyrro 1-3-propanoic acid or a salt thereof within 2 weeks after transarterial embolization to a hepatocellular carcinoma patient who has been treated with transarterial embolization.

4. A postoperative adjuvant chemotherapeutic agent comprising (Z)-5-[(1,2-dihydro-2-oxo-3H-indol-3-ylidene)methyl]2,4-dimethyl-1H-pyrro 1-3-propanoic acid or a salt thereof, which is used to initiate administration of
(Z)-5-[(1,2-dihydro-2-oxo-3H-indol-3-ylidene)methyl]2,4-dimethyl-1H-pyrro 1-3-propanoic acid or a salt thereof within 2 weeks after transarterial embolization to a hepatocellular carcinoma patient who has been treated with transarterial embolization for treatment of hepatocellular carcinoma.

5. The postoperative adjuvant chemotherapeutic agent of claim 4, wherein transarterial embolization is transarterial chemoembolization.

6. A kit including the postoperative adjuvant chemotherapeutic agent of claim 4 or 5 and instructions for use that specify initiation of administration of
(Z)-5-[(1,2-dihydro-2-oxo-3H-indol-3-ylidene)methyl]2,4-dimethyl-1H-pyrro 1-3-propanoic acid or a salt thereof within 2 weeks after transarterial embolization to a hepatocellular carcinoma patient who has been treated with transarterial embolization.

7. A method for treating hepatocellular carcinoma of a hepatocellular carcinoma patient who has been treated with transarterial embolization, which comprises initiating administration of an antitumor agent comprising (Z)-5-[(1,2-dihydro-2-oxo-3H-indol-3-ylidene)methyl]2,4-dimethyl-1H-pyrro 1-3-propanoic acid or a salt thereof to the hepatocellular carcinoma patient within 2 weeks after transarterial embolization.

8. The method of claim 7, wherein transarterial embolization is transarterial chemoembolization.

9. A method for treating hepatocellular carcinoma of a hepatocellular carcinoma patient who has been treated with transarterial embolization, which comprises administering a postoperative adjuvant chemotherapeutic agent comprising
(Z)-5-[(1,2-dihydro-2-oxo-3H-indol-3-ylidene)methyl]2,4-dimethyl-1H-pyrro 1-3-propanoic acid or a salt thereof to the hepatocellular carcinoma patient within 2 weeks after transarterial embolization.

10. The method of claim 9, wherein transarterial embolization is transarterial chemoembolization.

11. (Z)-5-[(1,2-dihydro-2-oxo-3H-indol-3-ylidene)methyl]2,4-dimethyl-1H-pyrrol-3-propanoic acid or a salt thereof, which is used for a hepatocellular carcinoma patient who has been treated with transarterial embolization within 2 weeks after transarterial embolization for treatment of hepatocellular carcinoma.

12. The
(Z)-5-[(1,2-dihydro-2-oxo-3H-indol-3-ylidene)methyl]2,4-dimethyl-1H-pyrro 1-3-propanoic acid or a salt thereof of claim 11, wherein transarterial embolization is transarterial chemoembolization.
